**Europäisches Patentamt**

**European Patent Office**

**Office européen des brevets**

(11) Publication number: **0 430 151 B1**

# EUROPEAN PATENT SPECIFICATION

(45) Date of publication of patent specification: **25.01.95** (51) Int. Cl.⁶: **C12Q 1/04**, C12M 1/14, C12M 1/38

(21) Application number: **90122587.0**

(22) Date of filing: **27.11.90**

(54) **Selective fungal medium.**

(30) Priority: **30.11.89 US 443612**

(43) Date of publication of application:
**05.06.91 Bulletin 91/23**

(45) Publication of the grant of the patent:
**25.01.95 Bulletin 95/04**

(84) Designated Contracting States:
**AT BE CH DE DK ES FR GB GR IT LI LU NL SE**

(56) References cited:
**EP-A- 0 107 070**
**WO-A-89/00204**
**CH-A- 614 466**
**US-A- 4 906 573**

(73) Proprietor: **Becton Dickinson and Company**
**One Becton Drive**
**Franklin Lakes,**
**New Jersey 07417-1880 (US)**

(72) Inventor: **Goldenbaum, Paul E., Ph.D.**
**732 Silver Creek Road**
**Baltimore, MD 21208 (US)**
Inventor: **Siddiqi, Salman, Ph.D.**
**15 Glencoe Manor Court**
**Sparks, MD 21152 (US)**

(74) Representative: **von Kreisler, Alek,**
**Dipl.-Chem. et al**
**Patentanwälte**
**von Kreisler-Selting-Werner**
**Postfach 10 22 41**
**D-50462 Köln (DE)**

Rank Xerox (UK) Business Services
(3.10/3.09/3.3.3)

## Description

FIELD OF THE INVENTION

The present invention relates to recovery and detection of yeasts, filamentous fungi and other clinically significant fungi from normally sterile body fluids. More particularly it relates to a liquid culture medium for use in recovering and detecting clinically significant fungi.

BACKGROUND OF THE INVENTION

Detection of mycological infection has become increasingly important in an age of immuno-compromised patients (e.g. kidney transplant, chemo-therapy, auto immune disease and acquired immune deficiency patients). Unfortunately, a single medium which is suitable for rapid growth and detection of a broad array of clinically significant fungi and is economical does not exist. An authoritative manual states "most fungal serologic tests (except cryptococcal antigen testing) are generally not helpful in making a diagnosis of fungal infections in the immunocompromised patient . . . a battery of appropriate media should be used since no single culture medium alone is adequate for the recovery of all fungal etiologic agents." Roberts, G.D. et al. "Detection and Recovery of Fungi in Clinical Specimens," Manual of Clinical Microbiology, 4th Ed., Lennette, E.H. et al., ed. (1985). Media recommended for the recovery of fungi from clinical specimens are (as recommended on page 510): Brain heart infusion (BHI) agar, Inhibitory mold agar, Sadhi agar, Yeast extract phosphate agar, BHI agar with 5 to 10% sheep blood, and various special media (i.e. Mycobiotic agar, Mycosel agar). These are all solid (agar containing) media. The composition of these media can be found in Manual of Clinical Microbiology, Chapter 111 (Culture Media). Other media used to grow fungi are: Sabouraud agar, Sabouraud dextrose agar, and Malt extract agar.

Almost all of the many remaining media described for use with fungi are specialized to allow the growth of select groups of fungi only, or are used for their ability to help identify certain fungi. These are therefore quite different from the media listed above which attempt to support the growth of the widest possible range of different types of fungi.

Virtually all culture media designed for the recovery, cultivation, growth and identification of fungi are solid or biphasic. The Isolator™ System (DuPont, Wilmington, Del) uses solid media. With this system, the sample must be transferred to a centrifuge tube and centrifuged. Thereafter a small volume of the centrifuged material is plated on a number of solid media. The Septi-Chek™ system (Roche Diagnostics, Nutley, NJ) is a biphasic system having a solid malt agar on a paddle which must be affixed to a bottle containing a broth medium after inoculation of the broth with the sample. The malt is optimized for fungi, but it is not selective. Another type of biphasic system is known generically as the Castaneda bottle. These systems are available from a number of manufacturers and contain both liquid and solid media together in a bottle. Biphasic systems suffer a significant drawback in that sufficient aeration of the liquid phase can not be acheived.

Solely liquid blood culture media are available which allow growth and detection of fungi, but these media are intended for growth and recovery of both bacteria and fungi. Where bacteria are present, rapid growth of the bacteria may mask fungi which grow more slowly.

Automated blood culture systems such as the Bactec® System (Becton Dickinson Diagnostic Instrument Systems, Towson, MD) are well suited for screening fluid samples for the presence of fungi. Each of the Bactec® systems comprises an instrument which incubates blood culture bottles containing a medium and sample and periodically tests the bottle for an increase in carbon dioxide indicating the presence of microorganisms. The Bactec 460® system uses culture media having $C^{14}$ enriched nutrients. Consumption of these nutrients results in production of gaseous $C^{14}O_2$ in a head space above the level of the culture medium in the bottle. The instrument periodically samples the head space gas and detects increased radioactivity. The Bactec NR-660® and Bactec NR-730® systems use non-radiometric media. In these systems growth of microorganisms is detected by measuring an increase in absorbance of near infra-red radiation associated with an increased concentration of $CO_2$.

Media used in the Bactec® systems will support growth of fungi, but are not well suited for growth and detection of fungi. They are primarily designed for growth of bacteria so that fungi do not optimally grow. Also the presence of fungi may be masked by overgrowth of any bacteria present. Finally the metabolism of blood in the sample may mask the presence of fungi for two reasons. First, blood cells themselves consume nutrients producing background levels of increased $CO_2$. While this background noise is acceptable when detecting much more rapidly growing bacteria, it is unacceptable when attempting to detect comparatively slow growing fungi. It is even more problematic when using a large blood sample (e.g.

10 ml) which is desirable when screening for fungemia. Secondly, blood inhibits fungal growth.

Further, WO 89/00204 discloses a growth medium for yeasts, which selectively inhibits procariotic microorganism, EP-A-0 107 070 discloses an antimicrobial factor deactivation system which can encompass anticoagulants and lysing agents (when desired to lyse blood) and CH-A-614466 discloses a method for identifying fungi and yeast in a liquid or solid culture medium by performing microscopic examination. However, none of the culture media disclosed in the above documents is feasible to selectively culture fungi.

Accordingly, a need exists for a liquid medium in which a broad array of clinically significant fungi will rapidly and selectively grow.

## SUMMARY OF THE INVENTION

The present invention is a liquid medium designed to promote the growth of various fungi while inhibiting the growth of non-fungal microorganisms. A sample of normally sterile body fluid (such as pleural, synovial, cerebral spinal, ascitic, etc.), or preferably blood, is added aseptically to the medium and the vial is then incubated at 30° to 35° C with agitation for a period of up to several weeks. The presence of viable fungi in the medium vial is indicated by visually inspecting the vial for increased turbidity, medium color changes, bulging septa, or other indications of organism growth, or by aseptically removing a small sample and performing a microscopic examination or a subculture to suitable solid media.

Preferably growth of fungi in the medium is detected with an automated blood culture systems such as the Bactec® system. For use in the Bactec® NR 660 and NR 730 the medium may be packaged in a 50 ml vial with gas above the fluid (head space gas) enriched with carbon dioxide of a specific and known concentration. Then growth of fungi may be detected by measuring increased levels of carbon dioxide.

Fungi which can be grown in the medium of the present invention range from "yeasts" through dimorphic, or filamentous forms. Typical examples include species of Candida, Torulopsis, Cryptococcus, Geotrichum, Saccharomyces, Aspergillus, and Histoplasma.

The liquid medium of the present invention is comprised of nitrogenous nutrients, one or more sugar selected from the group consisting of glucose, sucrose, and maltose, a blood anticoagulant, a source of ferric or ferrous iron, and an effective amount of one or more antimicrobials which inhibits growth of bacteria without inhibiting fungal growth. The medium differs from conventional media in that the ratio of sugar to nitrogenous nutrients is increased to a nominal 1:2, it includes chemicals to stimulate fungal growth (myo-inositol and ferric ammonium citrate), and it includes antimicrobials to inhibit bacterial growth. This unique combination produces two primary effects:

1. Optimal conditions for the growth of fungi in a fluid culture medium, and
2. Inhibition of non-fungal microorganisms.

The nitrogenous nutrients may be selected from those frequently used in media for bacteria such as pancreatic digest of casein, papaic digest of soy bean meal, pancreatic digest of gelatin, infusion of calf brain, infusion of beef heart, extract Of yeast autolysate, beef extract, malt extract, animal tissue extracts, and polypeptones. Preferably the nitrogenous nutrients comprise soybean-casein digest broth, brain-heart infusion broth, and extract of yeast autolysate.

The anticoagulant may be selected from the group consisting of sodium polyanetholsulfonate, sodium amylosulfate, sodium citrate, and ethylenediamine-tetraacetic acid. Preferably the anticoagulant is sodium polyanetholsulfonate because in addition to its function as an anticoagulant it serves to reduce the antimicrobial action of blood on fungi.

The ingredients in the medium which inhibit the growth of non-fungal microorganisms, or more specifically, bacteria, are the antimicrobials. Generally, an antimicrobial may be employed if it is not harmful to fungi and if it is stable in the medium or a period of several months or longer. The number of antimicrobials present is also variable from one to five or more. The selection and number present is based on the ability to inhibit the growth of the widest possible range of bacteria. Preferably a mixture of chloramphenicol and tobramycin is used. These two antimicrobials were selected for their combined ability to inhibit the growth of a very large number of both gram negative and gram positive bacteria.

The preferred medium includes a blood lysing agent. The presence of the lytic agent produces three effects. It eliminates the production of $CO_2$ from components of the blood itself, it lyses white cells thereby releasing any fungi which may have been engulfed, and it diminishes the inhibitory effect which blood has on the growth of many fungi.

The value of the first above effect is to lower the so called "background" of $CO_2$, or that $CO_2$ produced from sources other than the fungi. When volumes of blood in the range of 5 ml or greater are present, a high background would be produced unless the blood cells are lysed. By eliminating or lowering the blood background, a more favorable signal-to-noise condition is produced when the medium is used in the Bactec® system so that the instrument is able to detect the presence of fungal growth with greater certainty and speed.

The preferred lytic agent employed is saponin. Alternatively other lysing agents such as detergents, surfactants and enzymes. Typical examples include Triton X-100, various Tween and Brij detergents, and the proteolytic enzyme Rhozyme.

## DETAILED DESCRIPTION OF THE INVENTION

In the art of formulating media, concentrations of constituents are customarily expressed in weight per volume percentages (grams per 100 ml). In the description and claims that follow this convention is used. The fungal medium of the present invention comprises from 1.0 to 3.0 percent (weight/volume) nitrogenous nutrients, from 0.5 to 1.0 percent (weight/volume) of one or more sugars selected from the group consisting of glucose, sucrose, and maltose, from 0.01 to 0.125 percent (weight/volume) of a blood anticoagulant, a source of ferric or ferrous iron in an amount effective to stimulate fungal growth, and an effective amount of one or more antimicrobials which inhibit bacterial growth without affecting fungal growth. The ratio of nitrogenous nutrients to sugars falls in the range of from 1:1 to 6:1 and preferably from 2:1 to 6:1.

The nitrogneous nutrients conveniently comprise 1.5 percent (weight/volume) and are selected from the group consisting of pancreatic digest of casein, papaic digest of soy bean meal, pancreatic digest of gelatin, infusion of calf brain, infusion of beef heart, extract of yeast autolysate, beef extract, malt extract, animal tissue extracts, and polypeptones.

Preferably the nitrogneous nutrients comprise from 0.3 to 0.7 percent (weight/volume) soybean-casein digest broth, from 0.8 to 1.2 percent (weight/volume) brain-heart infusion broth, and from 0.02 to 0.6 percent (weight/volume) extract of yeast autolysate. Most preferably the nitrogenous nutrients comprise 0.5 percent (weight/volume) soybean-casein digest broth, 1.0 percent (weight/volume) brain-heart infusion broth, and 0.035 percent (weight/volume) extract of yeast autolysate. Each of these components is available from Becton Dickinson Microbiology Systems.

The preferred sugar component is a mixture of glucose and sucrose. Preferably from 0.5 to 1.0 percent (weight/volume) of a mixture of glucose and sucrose is used. The preferred mixture includes from 0.4 to 0.8 percent (weight/volume) sucrose and from 0.05 to 0.3 percent (weight/volume) glucose. In the most preferred formulation 0.6 percent (weight/volume) sucrose and 0.1 percent (weight/volume) glucose ae used.

The preferred anticoagulant is sodium polyanetholsulfonate. Preferably the fungal medium contains from 0.01 to 0.125 percent (weight/volume) anticoagulant. Most preferably 0.025 percent (weight/volume) sodium polyanetholsulfonate is used. Sodium polyanetholsulfonate is available commercially from Biosynth, Skokie, IL. Other suitable anticoagulants include sodium amylosulfate, sodium citrate, and ethylenediaminetetraacteic acid.

Iron is included to promote fungal growth. The iron is present as ferric or ferrous iron. Ferric ammonium sulfate in an amount of 0.0001 percent (weight/volume) is preferred. Alternatively any other organic or inorganic source of ferric or ferrous iron may be used. For example, ferric ammonium sulfate, ferric chloride and ferrous sulfate may be substituted.

Myo-inostol is a polyol alcohol that promotes growth of certain fungi, Cryptococcus neoformans in particular. It is preferably included at a level of 0.05 percent (weight/volume).

The antibiotics are present to inhibit growth of bacteria. While chloramphenicol and tobramycin are preferred, other antibiotics which do not inhibit fungi may be substituted. For example ciprofloxicin, norfloxicin or ofloxacin may be substituted for chloramphenicol and gentamicin or amikacin may be substituted for tobramycin. Alternatively mixtures of any of the foregoing may be used.

One embodiment of the medium includes an agent to lyse blood cells. Suitable lysing agents include saponin, Triton X-100 and various Tween and Brij detergents. Preferably saponin is used at a level of 0.1 to 0.4 percent (weight/volume). Most preferably saponin is present at a level of 0.24 percent (weight/volume).

A particularly preferred configuration of the medium of the present invention is suited for use in Bactec® automated blood culture systems. When the medium is to be used with a radiometric Bactec® system, a source of $^{14}$Carbon is included as taught by U.S. Patent No. 3,858,045. When the medium is to be used with a non-radiometric Bactec® System, the space over the medium within the bottle is filled with a mixture of $CO_2$ and air so that the equilibrated concentration of $CO_2$ in the bottle is from 1.0 to 3.5 percent.

When the medium is to be used with a conventional blood sample not exceeding 5 ml, a 50 ml bottle is filled with 30 ml of medium. When the fungal medium includes a blood lysing agent it can be used to test larger blood samples of up to 10 ml. With a larger blood sample, the time to detect the presence of fungi can be significantly reduced if a blood lysing agent is present. When a large blood sample will be tested, a 50 ml bottle should contain 25 ml of medium.

Further understanding of the fungal medium and its performance to rapidly detect the presence of fungi can be obtained from the following non-limiting examples.

EXPERIMENT 1

In this experiment, growth and detection of fourteen different strains of fungi were compared in several blood culture media. In each instance a bottle containing a medium was inoculated with a sample of banked human blood and with a known fungus. The media were the standard BACTEC® NR6A (non-radiometric, available from Becton Dickinson Diagnostic Instrument Systems) and two different configur- ations of the fungal medium of the present invention. In each instance the fungal medium contained:

0.5 percent (weight/volume) soybean-casein digest broth,

1.0 percent (weight/volume) brain-heart infusion broth,

0.035 percent (weight/volume) extract of yeast autolysate,

0.6 percent (weight/volume) sucrose,

0.1 percent (weight/volume) glucose,

0.05 percent (weight/volume) myo-inositol,

0.025 percent (weight/volume) sodium polyanetholesulfonate,

0.0001 percent (weight/volume) ferric ammonium citrate,

0.005 percent (weight/volume) chloramphenicol, and

0.001 percent (weight/volume) tobramycin.

The first set of fungal medium bottles (designated FM in Table 1) contained 30 ml of medium and a blood sample size of 4 ml. The fungal medium did not contain a lytic agent. The second set of fungal medium bottles (designated HBV-FM in Table 1) contained 25 ml of medium. These bottles were tested with blood samples of 4 ml and 10 ml. The fungal medium used in the second set of bottles contained 0.24 percent (weight/volume) saponin.

The size of the fungal inoculum was between 5 and 95 c.f.u. per vial in all cases. All vials were incubated at 35° ±1° C and were agitated on an orbital shaker at 280 ± 20 rpm for the first 48 hours of incubation. The vials were tested on a BACTEC® 660 instrument twice a day for the first two days and once a day thereafter for a total of seven days.

Vials were considered instrument positive if the Growth Value (GV) exceeded 20 or increased more than 10 over the previous reading.

In Table 1 TTD = Time To Detection (in days) on the BACTEC® instrument. GV = Growth Value for the vials when they became instrument positive (GV at the time of positivity). ND = No Detection (by GV on the BACTEC® 660), and + = subculture positive (a subculture to an agar plate showed that the vial contained living fungi, even though the growth was not detected on the BACTEC® instrument). All values shown are the averages of duplicate vials.

EP 0 430 151 B1

TABLE 1

| ORGANISM | MEDIUM | BLOOD VOLUME | TTD | GV |
|---|---|---|---|---|
| Candida albicans # 61 | 6A | 4ml | 1.0 | 25 |
| | FM | 4ml | 1.0 | 71 |
| | HBV-FM | 4ml | 1.0 | 29 |
| | HBV-FM | 10ml | 1.8 | 224 |
| Candida albicans # 399 | 6A | 4ml | 1.0 | 43 |
| | FM | 4ml | 1.0 | 168 |
| | HBV-FM | 4ml | 1.0 | 111 |
| | HBV-FM | 10ml | 1.0 | 78 |
| Candida albicans # 497 | 6A | 4ml | 1.0 | 37 |
| | FM | 4ml | 1.0 | 172 |
| | HBV-FM | 4ml | 1.0 | 72 |
| | HBV-FM | 10ml | 1.0 | 49 |
| Candida albicans # 10231 | 6A | 4ml | 1.0 | 30 |
| | FM | 4ml | 1.0 | 71 |
| | HBV-FM | 4ml | 1.0 | 31 |
| | HBV-FM | 10ml | 1.8 | 224 |
| Candida tropicalis # 229 | 6A | 4ml | 0.8 | 53 |
| | FM | 4ml | 0.8 | 223 |
| | HBV-FM | 4ml | 0.8 | 68 |
| | HBV-FM | 10ml | 0.8 | 84 |
| Candida tropicalis # 407 | 6A | 4ml | 1.0 | 49 |
| | FM | 4ml | 1.0 | 112 |
| | HBV-FM | 4ml | 1.0 | 66 |
| | HBV-FM | 10ml | 1.0 | 87 |
| Candida parapsilosis # 228 | 6A | 4ml | 1.8 | 54 |
| | FM | 4ml | 1.8 | 43 |
| | HBV-FM | 4ml | 1.8 | 160 |
| | HBV-FM | 10ml | 1.8 | 115 |

| ORGANISM | MEDIUM | BLOOD VOLUME | TTD | GV |
|---|---|---|---|---|
| Candida rugosa | 6A | 4ml | ND | + |
| | FM | 4ml | 3.2 | 27 |
| | HBV-FM | 4ml | 3.0 | 22 |
| | HBV-FM | 10ml | 4.0 | 30 |
| Torulopsis glabrata # 156 | 6A | 4ml | 1.8 | 34 |
| | FM | 4ml | 1.8 | 25 |
| | HBV-FM | 4ml | 1.8 | 185 |
| | HBV-FM | 10ml | 1.8 | 183 |
| Torulopsis glabrata # 231 | 6A | 4ml | 1.8 | 42 |
| | FM | 4ml | 1.8 | 130 |
| | HBV-FM | 4ml | 1.0 | 36 |
| | HBV-FM | 10ml | 1.8 | 152 |
| Saccharomyces cerevisae # 414 | 6A | 4ml | 1.0 | 43 |
| | FM | 4ml | 1.0 | 223 |
| | HBV-FM | 4ml | 1.0 | 173 |
| | HBV-FM | 10ml | 1.0 | 155 |
| Cryptococcus neoformans # 369 | 6A | 4ml | 5.0 | 18 |
| | FM | 4ml | 4.0 | 35 |
| | HBV-FM | 4ml | 3.0 | 20 |
| | HBV-FM | 10ml | 3.0 | 30 |
| Cryptococcus neoformans # 695 | 6A | 4ml | ND | + |
| | FM | 4ml | 4.0 | 26 |
| | HBV-FM | 4ml | 3.0 | 29 |
| | HBV-FM | 10ml | 3.0 | 33 |
| Geotrichum sp. # 230 | 6A | 4ml | 1.0 | 48 |
| | FM | 4ml | 0.8 | 24 |
| | HBV-FM | 4ml | 0.8 | 50 |
| | HBV-FM | 10ml | 1.0 | 95 |

The above experiment shows that the FM and HBV-FM media are better than the standard BACTEC® NR6A medium in terms of higher GVs and/or faster TTDs for most of the fungi tested. These organisms were used because they represent the fungi most commonly isolated from clinical blood samples.

EXPERIMENT 2

The fungal medium configuration (as described above) was tested for TTD against regular BACTEC® NR6A blood culture medium and two (2) different biphasic culture media commonly used for blood cultures. As described earlier, biphasic media are generally considered better than all liquid media for the culturing

7

of fungi from blood samples. The two biphasic bottles were:

1. Castaneda "S", manufactured by Diagnostics Pasteur containing Sabouraud dextrose agar and broth. Non- selective (no antimicrobials present)

2. Hemoline Sabouraud Diphasique, manufactured by bioMerieux, containing both agar and broth. Is selective for fungi, for it contains 0.01% gentamicin to limit the growth of bacteria.

All determinations were conducted in duplicate. Inocula were between 5 and 50 c.f.u total per bottle or vial. Human blood was added, 5ml in BACTEC® NR6A and fungal medium (FM) and 10 ml in the biphasic bottles as called for by the manufacturers. All cultures were incubated at 35° ± 1° C and the BACTEC® NR6A and fungal media were agitated by orbital shaking at 280 ± 20 rpm for the first 48 hours. Positivity on the BACTEC® NR6A and the fungal medium was determined by reading on a BACTEC® 660 instrument using a threshold of 20 and a delta (change from one reading to the next) GV of 10. For the biphasic bottles, positivity was based on visual observation of colonies growing on the agar surface or turbidity in the broth, or both.

Organisms tested included three strains of bacteria (last on Table 2) to show the selective function of the FM and Hemoline media. In Table 2, TTD (Time To Detection) is given in hours. NG = No Growth present at end of testing after seven days.

TABLE 2

| ORGANISM | TTD IN HOURS MEDIA | | | |
|---|---|---|---|---|
| | NR6A | FM | HEMOLINE | CASTANEDA |
| Candida albicans #61 | 42 | 42 | 42 | 42 |
| Candida albicans #399 | 25 | 25 | 42 | 42 |
| Candida albicans #497 | 25 | 25 | 66 | 66 |
| Candida parapsilosis #491 | 42 | 42 | 42 | 42 |
| Candida tropicalis #407 | 25 | 25 | 42 | 42 |
| Torulopsis glabrata #156 | 90 | 42 | 42 | 42 |
| Torulopsis glabrata #231 | 66 | 42 | 42 | 42 |
| Geotrichum sp. #230 | 25 | 25 | 42 | 42 |
| Cryptococcus neoformans #691 | 90 | 90 | 114 | 102 |
| Cryptococcus neoformans #696 | 42 | 42 | 90 | 102 |
| Pseudomonas aeruginosa #27853 | 25 | NG | NG | 42 |
| Staphylococcus aureus #29213 | 18 | NG | NG | 25 |
| Escherichia coli #25922 | 18 | NG | NG | 25 |

It can be seen from these data that the FM is generally faster than the biphasic media in detecting the representative fungi tested. The FM also prevented growth of bacteria (last three organisms) which is an advantage not present in the regular BACTEC® NR6A or in the Castaneda biphasic bottle.

**Claims**

**1.** A liquid medium for selectively culturing fungi, comprising:
(a) from 1.0 to 3.0 percent (weight/volume) nitrogenous nutrients,
(b) from 0.5 to 1.0 percent (weight/volume) of one or more sugars,
(c) from 0.01 to 0.125 percent (weight/volume) of a blood anticoagulant,
(d) a source of ferric or ferrous iron in an amount effective to stimulate fungal growth, and
(e) an effective amount of one or more antimicrobials which inhibit bacterial growth without affecting fungal growth,
wherein the ratio of nitrogenous nutrients to sugar is from 1:1 to 6:1.

**2.** The liquid medium of Claim 1 wherein said nitrogenous nutrients comprise a mixture of one or more nitrogenous nutrients selected from the group consisting of pancreatic digest of casein, papaic digest of soy bean meal, pancreatic digest of gelatin, infusion of beef heart, extract of yeast autolysate, beef extract, malt extract, animal tissue extract and polypeptone.

3. The liquid medium of Claim 1 wherein said sugars comprise a mixture of one or more sugars selected from the group consisting of glucose, sucrose and maltose.

4. The liquid medium of Claim 1 wherein said blood anticoagulant is selected from the group consisting of sodium polyanetholsulfonate, sodium amylosulfate, sodium citrate, and ethylenediamine-tetraacetic acid.

5. The liquid medium of Claim 1 wherein said source of ferric or ferrous iron is selected from the group consisting of ferric ammonium sulfate, ferric chloride and ferrous sulfate.

6. The liquid medium of Claim 4 wherein said source of ferric or ferrous iron is ferric ammonium sulfate.

7. The liquid medium of Claim 1 wherein said antimicrobials comprise a mixture of one or more antimicrobials selected from the group consisting of chloramphenicol and tobramycin.

8. The liquid medium of Claim 1 further comprising an effective amount of myo-inositol.

9. A method for selectively culturing fungi comprising:
    (a) adding a sample of sterile body fluid to a liquid medium comprising:
        (i) nitrogenous nutrients;
        (ii) one or more sugars;
        (iii) a blood anticoagulant;
        (iv) a source of a ferric or ferrous iron;
        (v) one or more antimicrobials; and
        (vi) a blood lysing agent,
        (vii) a myo-inositol.
    (b) incubating the mixture from 30° to 35°C and agitating for 2 weeks;
    (c) visually inspecting said mixture for indications of organism growth; and
    (d) performing a microscopic examination of said mixture for presence of viable fungi.

10. A method for selectively culturing fungi comprising:
    (a) adding a sample of sterile body fluid to a liquid medium comprising:
        (i) 0.5% (w/v) soybean-casein digest broth;
        (ii) 1.0% (w/v) brain-heart infusion broth;
        (iii) 0.035% (w/v) extract of yeast auto- lysate;
        (iv) 0.6% (w/v) sucrose;
        (v) 0.1% (w/v) glucose;
        (vi) 0.025% (w/v) sodium polyanethol- sulfonate;
        (vii) 0.0001 % (w/v) ferric ammonium sulfate;
        (viii) 0.05% (w/v) myo-inositol;
        (ix) 0.005% (w/v) chloramphenicol;
        (x) 0.001% (w/v) tobramycin; and
        (xi) 0.24% (w/v) saponin,
    (b) incubating the mixture from 30° to 35°C and agitating for 2 weeks;
    (c) visually inspecting said mixture for indications of organism growth; and
    (d) performing a microscopic examination of said mixture for presence of viable fungi.

**Patentansprüche**

1. Flüssiges Medium zur Kultivierung von Fungi, umfassend
    (a) 1,0 bis 3,0 % (Gew./Vol.) stickstoffhaltige Nährstoffe,
    (b) 0,5 bis 1,0 % (Gew./Vol.) eines Zuckers oder mehrere Zucker,
    (c) 0,01 bis 0,125 % (Gew./Vol.) eines Blut-Antikoagulationsmittels,
    (d) eine Quelle für Eisen(III)- oder Eisen(II)-Ionen in einer das Pilzwachstum fördernden Menge und
    (e) eine wirksame Menge eines oder mehrerer antimikrobieller Mittel zur Hemmung des Bakterienwachstums, ohne das Pilzwachstum zu beeinflussen,
    wobei das Verhältnis der stickstoffhaltigen Nährstoffe zu dem bzw. den Zucker(n) 1 : 1 bis 6 : 1 beträgt.

**2.** Flüssiges Medium nach Anspruch 1, worin die stickstoffhaltigen Nährstoffe eine Mischung aus einem oder mehreren stickstoffhaltigen Nährstoff(en) umfassen, die aus der aus dem pankreatischen Aufschluß von Casein, dem Papain-Aufschluß von Sojabohnenmehl, dem pankreatischen Aufschluß von Gelatine, einer Rinderherz-Infusion, dem Extrakt von Hefe-Autolysat, Rindfleischextrakt, Malzextrakt, tierischem Gewebeextrakt und Polypepton bestehenden Gruppe ausgewählt sind.

**3.** Flüssiges Medium nach Anspruch 1, worin die Zucker eine Mischung aus einem oder mehreren Zucker(n) umfassen, die aus der aus Glucose, Saccharose und Maltose bestehenden Gruppe ausgewählt sind.

**4.** Flüssiges Medium nach Anspruch 1, worin das Blut-Antikoagulationsmittel aus der aus Natriumpolyanetholsulfonat, Natriumamylosulfat, Natriumcitrat und Ethylendiamintetraessigsäure bestehenden Gruppe ausgewählt ist.

**5.** Flüssiges Medium nach Anspruch 1, worin die Quelle für Eisen(III)- oder Eisen(II)-Ionen aus der aus Eisen(III)-ammoniumsulfat, Eisen(III)-chlorid und Eisen(II)-sulfat bestehenden Gruppe ausgewählt ist.

**6.** Flüssiges Medium nach Anspruch 4, worin die Quelle für Eisen(III)- oder Eisen(II)-Ionen Eisen(III)-ammoniumsulfat ist.

**7.** Flüssiges Medium nach Anspruch 1, worin die antimikrobiellen Mittel eine Mischung aus einem oder mehreren antimikrobiellen Mittel(n) umfaßt, die aus der aus Chloramphenicol und Tobramycin bestehenden Gruppe ausgewählt sind.

**8.** Flüssiges Medium nach Anspruch 1, weiterhin umfassend eine wirksame Menge *myo*-Inosit.

**9.** Verfahren zum selektiven Kultivierung von Fungi, umfassend
(a) das Hinzufügen einer Probe einer sterilen Körperflüssigkeit zu einem flüssigen Medium, das
  (I) stickstoffhaltige Nährstoffe,
  (II) einen oder mehrere Zucker,
  (III) ein Blut-Antikoagulationsmittel,
  (IV) eine Quelle für Eisen(III)- oder Eisen(II)-Ionen,
  (V) ein oder mehrere antimikrobielle Mittel und
  (VI) ein Blut lysierendes Mittel,
  (VII) *myo*-Inosit
umfaßt,
(b) das Inkubieren der Mischung bei 30 °C bis 35 °C unter Rühren während 2 Wochen,
(c) das visuelle Überwachen der Mischung auf Anzeichen eines Wachstums von Organismen, und
(d) das Durchführen einer mirkoskopischen Untersuchung der Mischung auf die Anwesenheit lebensfähiger Fungi.

**10.** Verfahren zur selektiven Kultivierung von Fungi, umfassend
(a) das Hinzufügen einer Probe einer sterilen Körperflüssigkeit zu einem flüssigen Medium, das
  (I) 0,5 % (Gew./Vol.) Sojabohnen-Casein-Aufschlußbrühe,
  (II) 1,0 % (Gew./Vol.) Gehirn-Herz-Infusionsbrühe,
  (III) 0,035 % (Gew./Vol.) Extrakt von Hefe-Autolysat,
  (IV) 0,6 % (Gew./Vol.) Saccharose,
  (V) 0,1 % (Gew./Vol.) Glucose,
  (VI) 0,025 % (Gew./Vol.) Natriumpolyanetholsulfonat,
  (VII) 0,0001 % (Gew./Vol.) Eisen(III)-ammoniumsulfat,
  (VIII) 0,05 % (Gew./Vol.) *myo*-Inosit,
  (IX) 0,005 % (Gew./Vol.) Chloramphenicol,
  (X) 0,001 % (Gew./Vol.) Tobramycin und
  (XI) 0,24 % (Gew./Vol.) Saponin,
umfaßt,
(b) das Inkubieren der Mischung bei 30 °C bis 35 °C unter Rühren während 2 Wochen,
(c) das visuelle Überwachen der Mischung auf Anzeichen eines Wachstums von Organismen, und

(d) das Durchführen einer mirkoskopischen Untersuchung der Mischung auf die Anwesenheit lebensfähiger Fungi.

**Revendications**

1. Milieu liquide pour la culture sélective de champignons, comprenant :
   (a) de 1,0 à 3,0 % (masse/volume) de nutriments azotés,
   (b) de 0,5 à 1,0 % (masse/volume) d'un ou plusieurs sucres,
   (c) de 0,01 à 0,125 % (masse/volume) d'un anticoagulant du sang,
   (d) une source de fer ferrique ou ferreux en une quantité suffisante pour stimuler la croissance fongique, et
   (e) une quantité efficace d'un ou plusieurs agents antimicrobiens qui inhibent la croissance bactérienne sans affecter la croissance fongique,
   où le rapport des nutriments azotés au sucre est de 1:1 à 6:1.

2. Milieu liquide selon la revendication 1, dans lequel les nutriments azotés comprennent un mélange d'un ou plusieurs nutriments azotés choisis parmi l'ensemble comprenant le produit de la digestion pancréatique de la caséine, le produit de la digestion, par la papaïne, de la farine de soja, le produit de la digestion pancréatique de la gélatine, une infusion de coeur de boeuf, un extrait d'un autolysat de levure, un extrait de boeuf, un extrait de malt, un extrait de tissu animal et la polypeptone.

3. Milieu liquide selon la revendication 1, dans lequel les sucres comprennent un mélange d'un ou plusieurs sucres choisis parmi l'ensemble comprenant le glucose, le saccharose et le maltose.

4. Milieu liquide selon la revendication 1, dans lequel l'anticoagulant du sang est choisi parmi l'ensemble comprenant le polyanetholsulfonate de sodium, l'amylosulfate de sodium, le citrate de sodium et l'acide éthylènediamine-tétraacétique.

5. Milieu liquide selon la revendication 1, dans lequel la source de fer ferrique ou ferreux est choisie parmi l'ensemble comprenant le sulfate d'ammonium ferrique, le chlorure ferrique et le sulfate ferreux.

6. Milieu liquide selon la revendication 4, dans lequel la source de fer ferrique ou ferreux est le sulfate d'ammonium ferrique.

7. Milieu liquide selon la revendication 1, dans lequel les agents antimicrobiens comprennent un mélange d'un ou plusieurs agents antimicrobiens choisis parmi l'ensemble comprenant le chloramphénicol et la tobramycine.

8. Milieu liquide selon la revendication 1, qui comprend en outre une quantité efficace de myo-inositol.

9. Procédé de culture sélective de champignons, qui consiste :
   (a) à ajouter un échantillon d'un fluide corporel stérile à un milieu liquide comprenant :
      (i) des nutriments azotés,
      (ii) un ou plusieurs sucres,
      (iii) un anticoagulant du sang,
      (iv) une source de fer ferrique ou ferreux,
      (v) un ou plusieurs agents antimicrobiens, et
      (vi) un agent de lyse du sang,
      (vii) un myo-inositol,
   (b) à incuber le mélange de 30 à 35°C et à l'agiter pendant 2 semaines ;
   (c) à procéder à un contrôle visuel du mélange pour rechercher les signes de croissance d'organismes ; et
   (d) à effectuer un examen au microscope de ce mélange pour déterminer la présence de champignons viables.

10. Procédé de culture sélective de champignons, qui consiste :
    (a) à ajouter un échantillon d'un fluide corporel stérile à un milieu liquide comprenant :

(i) 0,5 % (m/v) d'un bouillon de digestion de soja-caséine,

(ii) 1,0 % (m/v) d'un bouillon d'infusion cerveau-coeur ;

(iii) 0,035 % (m/v) d'un extrait d'un auto-lysat de levure ;

(iv) 0,6 % (m/v) de saccharose ;

(v) 0,1 % (m/v) de glucose ;

(vi) 0,025 % (m/v) de polyanethol-sulfonate de sodium ;

(vii) 0,0001 % (m/v) de sulfate d'ammonium ferrique ;

(viii) 0,05 % (m/v) de myo-inositol ;

(ix) 0,005 % (m/v) de chloramphénicol ;

(x) 0,001 % (m/v) de tobramycine ; et

(xi) 0,24 % (m/v) de saponine,

(b) à incuber le mélange de 30 à 35°C et à l'agiter pendant 2 semaines ;

(c) à procéder à un contrôle visuel du mélange pour rechercher les signes de croissance d'organismes ; et

(d) à effectuer un examen au microscope de ce mélange pour déterminer la présence de champignons viables.